# EUROPÄISCHE PATENTSCHRIFT

(11) **EP 0 464 169 B1**
(45) Veröffentlichungstag und Bekanntmachung des Hinweises auf die Patenterteilung: **27.03.1996**
(21) Anmeldenummer: 91902236.8
(22) Anmeldetag: 22.01.1991
(51) Int. Cl.: A61M 5/175

(54) **VORRICHTUNG, DIE INSBESONDERE ALS MEDIZINISCHES INFUSIONS- ODER TRANSFUSIONSBESTECK VERWENDBAR IST**
DEVICE, IN PARTICULAR MEDICAL SET FOR INFUSION OR TRANSFUSION
DISPOSITIF UTILISABLE NOTAMMENT COMME TROUSSE MEDICALE POUR PERFUSION OU TRANSFUSION

(30) Priorität: 25.01.1990 DE 4002089
(43) Veröffentlichungstag der Anmeldung: 08.01.1992
(73) Patentinhaber: Forberg, Hans-Jürgen, D-23738 Damlos (DE)
(72) Erfinder: Forberg, Hans-Jürgen, D-23738 Damlos (DE)
(74) Vertreter: Vollmann, Heiko, Dipl.-Ing.
(86) Internationale Anmeldenummer: DE9100066
(87) Internationale Veröffentlichungsnummer: WO9111211

(56) Entgegenhaltungen:
- DE-A- 2 452 117
- DE-C- 2 745 317
- US-A- 4 378 013

## Beschreibung

Die Erfindung geht aus Von einer Vorrichtung gemäß dem Oberbegriff des Patentanspruches 1.

Eine derartige Vorrichtung ist in der DE-C-27 45 217 beschrieben. Sie umfaßt einen zylindrischen Kern, eine zylindrische Hülse aus flexiblem Material, die den Kern wenigstens auf seiner gesamten Länge mit radialem Abstand umgibt, und einen auf der Hülse sitzenden, diese umfangsmäßig radial zusammendrückenden Einstellring, der in Längsrichtung auf der Hülse verschiebbar ist. Der Kern hat in seinem Einlaufabschnitt einen axialen Einlaufkanal, der in radialen Auslaufkanälen endet, die wiederum in eine Umfangsnut des Kernes einmünden. Die Mantelfläche des Kernes ist mit einer wendelförmigen Nut versehen, die etwa im Bereich der Umfangsnut beginnt und kurz vor dem anderen und geschlossenen Ende des Kernes endet. Die wendelförmige Nut hat einen sich in Durchflußrichtung der Vorrichtung stetig verringernden Strömungsquerschnitt. Zur Einstellung des tatsächlichen Durchlaßquerschnittes der Vorrichtung wird der Einstellring axial verschoben und an der gewünschten Stelle des Kernes unter Anpressung der flexiblen Hülse an diesen auf der Hülse positioniert, so daß die wendelförmige Nut an dieser Stelle abgedeckt und nur an dieser Stelle ein Durchfluß möglich ist.

Eine weitere Vorrichtung ist in der US-A-43 78 013 bekannt. Bei dieser Vorrichtung wird für die Regulierung der Durchflußmenge eine Einrichtung verwendet, die geeignet ist, den Durchflußquerschnitt der Schlauchleitung einstellbar zu verändern. Diese Einrichtung besteht aus einer seitlichen Drehhandhabe mit einem Druckstift, der auf ein Regulierschlauchstück gedrückt wird, wenn die Handhabe gedreht wird. Eine ähnliche Vorrichtung ist in der DE-A-24 52 117 beschrieben. Die Abflußgeschwindigkeit der Infusionsmenge wird hier durch zwei mittels einer Drehhandhabe axial ineinander verstellbare Konen bewirkt. Den Konen ist ein Filter vorgeschaltet.

Beim praktischen Gebrauch hat sich jedoch gezeigt, daß die ordnungsgemäße Funktion der bekannten Einrichtungen durch die Elastizität des Schlauchstückes und dessen Durchmesser- und Wandstärkentoleranz negativ beeinflußt wird. Dabei kann das Elastizitätsverhalten sowohl durch Alterung als auch durch Verwendung unterschiedlicher Schlauchmaterialien unterschiedlcih sein, so daß sich Schläuche auch bei gleichem Durchmesser und gleicher Wandstärke in derselben Reguliereinrichtung sehr unterschiedlich verhalten, was auch einen negativen Einfluß auf die Konstanz der Durchflußrate hat. Die Verwendung eines Filters vor der Regelstelle der Vorrichtung beeinflußt die Durchflußrate ebenfalls negativ, weil dadurch ein gleichmäßiger Durchfluß nicht gewährleistet ist.

Die Aufgabe der Erfindung besteht in der Verbesserung der einleitend angeführten Vorrichtung dahingehend, eine vereinfachte und leicht montierbare Reguliereinrichtung vorzuschlagen, bei der die eingestellte Durchflußrate unabhängig von den genannten Schlauchparametern oder anderen Teilen sicher konstant gehalten werden kann. Darüber hinaus soll der Fertigungsaufwand für eine solche Einrichtung minimiert werden.

Diese Aufgabe wird erfindungsgemäß durch die Merkmale des Patentanspruches 1 gelöst.

Die damit erzielbaren Vorteile bestehen insbesondere darin, daß die Parameter des an die Vorrichtung angeschlossenen Schlauches, und zwar an dem verbundenen Anschlußstutzen, weitgehend ausgeschaltet sind, so daß eine eingestellte Durchflußrate an Infusions- oder Transfusionsmittel konstant eingehalten wird. Das ist darauf zurückzuführen, daß der Schlauchanteil des den Durchströmquerschnitt des nutartigen Ablaufkanales umgrenzendert Umfanges auf ein Minimum reduziert ist. Es weiteren ist die Reguliereinrichtung von einfachem Aufbau und kostengünstig herzustellen.

Besondere Feinfühligkeit der Einstellung kann dabei dadurch erreicht werden, daß das die Reguliermutter führende Gewinde des Gewindestutzens lediglich an der Mantelfläche derjenigen Segmente angeordnet ist, die nicht mit einem Reguliernoppen versehen sind, und dadurch, daß die Längsnuten einen V-förmig sich nach außen erweiternden Querschnitt aufweisen und die Reguliernoppen in die zugehörige Längsnut eintauchbar ausgebildet sind.

Vorteilhafterweise kann die Reguliermutter dadurch unverlierbar gemacht werden, daß der Gewindestutzen und/oder die Reguliermutter mit Mitteln versehen sind, die das Abdrehen der letzteren verhindert, zumindest aber erschwert.

Die erfindungsgemäße Vorrichtung ist nachstehend anhand eines in der anliegenden Zeichnung dargestellten Ausführungsbeispieles naher erläutert. Es zeigen:
- Fig. 1: einen Längsschnitt durch das Unterteil einer erfindungsgemäß ausgestalteten Vorrichtung nach der Linie I-I in Fig. 2,
- Figur 2: einen Querschnitt durch das Unterteil nach Figur 1 nach der Linie II-II in Fig. 1.

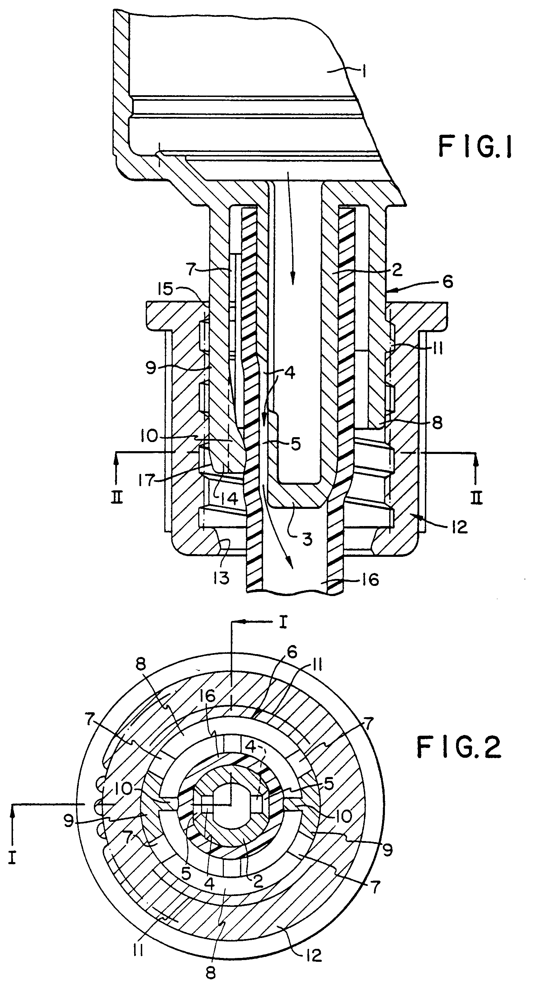

Wie insbesondere aus Figur 1 ersichtlich ist, ist eine nur teilweise gezeigte Tropfkammer 1 an ihrem Unterende mit einem rohrförmigen Anschlußstutzen 2 versehen, der an seinem unteren Stirnende 3 geschlossen ausgeführt ist. Der Stutzen 2 weist wenigstens einen seitlichen Durchbruch 4 auf. Figur 2 zeigt hier zwei einander gegenüberliegende Durchbrüche auf, die in jeweils eine zu dem Stirnende 3 hin offene Längsnut 5 V-förmigen Querschnitts münden, die in der Mantelfläche des Stutzens 2 angeordnet sind. Der Stutzen 2 ist von einem rohrförmigen Gewindestutzen 6 mit radialem Abstand umgeben, der durch axial verlaufende Teilungsfugen 7 (siehe Figur 2) in Segmente 8 und 9 aufgeteilt ist. Die Teilung ist dabei so gewählt, daß jeweils einer Längsnut 5 ein Segment 9 gegenüberliegt. Diese Segmente 9 sind jeweils mit einem jeweils einer Längsnut 5 radial gegenüberstehenden Reguliernoppen 10 versehen. Das Gewinde 11 des Gewindestutzens 6 ist im Bereich der Segmente 9 unterbrochen. Der Gewindestutzen 6 dient der Aufnahme einer Reguliermutter 12, die einen Innenkonus 13 aufweist, der mit dem Stirnende 14 der Segmente 9 in Kontakt treten kann.

Das vorstehend beschriebene Gerät funktioniert folgendermaßen:
Bei der Montage des Tropfkammerunterteils mit der Reguliermutter wird die Reguliermutter so aufgedrückt, daß der für die Unverlierbarkeit zuständige Innenwulst über das Gewindesegment schnappt. Damit ist die Reguliermutter in der vor der Montage des Infusionsschlauches gewünschten Position. In dieser Stellung ist die maximale Durchflußgeschwindigkeit der Infusionslösung erreicht. In dieser Stellung der Reguliermutter 12 wird eine Schlauchleitung 16 durch diese hindurch auf den Anschlußstutzen 2 aufgeschoben. Die Schlauchleitung 16 überdeckt dabei die Längsnuten 5, ohne jedoch deren Querschnitt zuzusetzen. Damit kann die Vorrichtung bereits eingesetzt werden. Die Regulierung der Durchflußmenge durch die Schlauchleitung erfolgt durch Aufschrauben der Reguliermutter 12 auf den Gewindestutzen 6, was bewirkt, daß die Stirnenden 14 der Segmente 9 in den Innenkonus 13 eintreten und somit radial nach innen ausgelenkt werden. Dadurch nähern sich die an diesen Segmenten 9 befindlichen Reguliernoppen 10 der Außenseite der Schlauchleitung 16 und drücken diese schließlich in die unter den Reguliernoppen 10 befindlichen Längsnuten 5. Dieser Vorgang kann bis zum vollständigen Ausfüllen des Querschnittes der Längsnuten 5 durch das Schlauchmaterial fortgeführt werden, so daß eine Verengung des Durchflußquerschnittes bis zu einem vollständigen Verschließen der Schlauchleitung erfolgen kann. Für eine bessere Einpassung der Stirnenden 14 der Segmente 9 in den Innenkonus 13 der Mutter können die Segmente 9 in ihrem Endbereich mit einer umlaufenden Abschrägung 17 versehen sein.

## Patentansprüche

1. Vorrichtung, die insbesondere als medizinisches Infusions- oder Transfusionsgerät verwendbar ist, mit einer eine Flüssigkeit enthaltenden Tropfkammer (1) mit einem Anschlußstutzen (2) zum Befestigen einer Schlauchleitung (16) und mit einer Einrichtung zum Regulieren der die Schlauchleitung durchfließenden Flüssigkeitsmenge als fester Bestandteil der Tropfkammer (1), wobei der Anschlußstutzen (2) an seinem unteren Stirnende (3) geschlossen ausgeführt und mit mindestens einem radialen Durchbruch (4) ausgestattet ist, dadurch gekennzeichnet, daß der Durchbruch (4) jeweils in eine zu dem Stirnende (3) hin offene Längsnut (5) in der Mantelfläche des Anschlußstutzens (2) mündet, daß der Anschlußstutzen (2) von einem rohrförmigen Gewindestutzen (6) mit einer Reguliermutter (12) umgeben ist, der durch axial verlaufende Teilungsfugen (7) in Segmente (8, 9) aufgeteilt ist, daß eine der Anzahl der Längsnuten (5) entsprechende Anzahl von Segmenten (9) radial federnd ausgebildet und mit einem jeweils einer Längsnut (5) radial gegenüberstehenden Reguliernoppen (10) versehen ist und daß an dem freien Ende (14) jedes dieser Segmente (9) ein an der Reguliermutter (12) befindlicher Innenkonus (13) zur Anlage bringbar ist, derart, daß die Reguliernoppen (10) der Segmente (9) einen Wandteil der Schlauchleitung (16) in die Längsnuten (5) des Anschlußstutzens (2) drücken.

2. Vorrichtung nach Anspruch 1, dadurch gekennzeichnet, daß das die Reguliermutter (12) führende Gewinde des Gewindestutzens (6) lediglich an der äußeren Mantelfläche solcher Segmente (9) angeordnet ist, die nicht mit einem Reguliernoppen (10) versehen sind.

3. Vorrichtung nach Anspruch 1 oder 2, dadurch gekennzeichnet, daß die Längsnuten (5) einen sich nach außen V-förmig erweiternden Querschnitt aufweisen und daß die Reguliernoppen (10) in ihre zugehörige Längsnut (5) einbringbar ausgebildet sind.

4. Vorrichtung nach Anspruch 2 oder 3, dadurch gekennzeichnet, daß der Gewindestutzen (6) und/oder die Reguliermutter (12) mit Mitteln (15) versehen sind, die das vollständige Abdrehen der Reguliermutter (12) von dem Gewindestutzen zumindest erschweren.

## Claims

1. A device, in particular for use as a medical infusion or transfusion apparatus, comprising a drip chamber (1) holding a fluid, and having a connection piece (2) for attaching a hose pipe (16), and a device, as a fixed component of the drip chamber (1), for regulating the quantity of fluid flowing through the hose pipe, said connection piece (2) being closed at its lower end face (3) and provided with at least one radial opening (4), characterised in that each opening (4) runs towards the end face (3) into an open longitudinal groove (5) in the lateral surface of the connection piece (2), that the connection piece (2) is surrounded by a tube-shaped screw connection (6) with a regulating nut (12), said screw connection being divided into segments (8, 9) by axial running separating gaps (7), that a number of segments (9) corresponding to the number of longitudinal grooves (5) are radially sprung and provided each with a regulating nap (10) radially opposite longitudinal groove (5), and that an inner cone (13) located on the regulating nut (12) can be brought into contact with the free end (14) of each of these segments (9) in a manner such that the regulating naps (10) of the segments (9) press a part of the wall of the hose pipe (16) into the longitudinal grooves (5) of the connection piece (2).

2. A device according to claim 1, characterised in that the thread of the screw connection (6) guiding the regulating nut (12) is arranged only on the outer lateral surface of those segments (9) which are not provided with regulating naps.

3. A device acording to claims 1 or 2 characterised in that the longitudinal grooves (5) comprise a V-shaped outwardly extending cross-section and that the regulating naps (10) can be placed into their accompanying longitudinal groove (5).

4. A device according to claim 2 or 3 characterised in that the screw connection (6) and/or the regulating nut (12) are provided with means (15) which at least impedes the twisting off of the regulating nut (12).

## Revendications

1. Dispositif, utilisable notamment comme appareillage médical de perfusion ou de transfusion, avec un compartiment compte-gouttes (1) qui contient un liquide et comporte un embout de raccordement (2) pour la fixation d'un conduit souple (16), et avec un dispositif pour régler la quantité de liquide s'écoulant à travers le conduit souple, comme élément fixe du compartiment compte-gouttes (1), l'embout de raccordement (2) étant fermé à son extrémité frontale inférieure (3) et étant muni d'au moins un perçage radial (4), caractérisé en ce que le perçage (4) débouche dans une rainure longitudinale (5), ouverte vers l'extrémité frontale (3) et ménagée dans la surface latérale de l'embout de raccordement (2), en ce que l'embout de raccordement (2) est entouré par un embout fileté (6) de forme tubulaire muni d'un écrou de réglage (12), embout fileté qui est divisé en segments (8, 9) par des joints de séparation (7) s'étendant dans le sens axial, en ce qu'un nombre de segments (9), qui correspond au nombre de rainures longitudinales (5), est formé avec une élasticité radiale et est pourvu d'un téton de réglage (10) radialement en face de chaque rainure longitudinale (5) et en ce qu'un cône intérieur (13), qui se trouve sur l'écrou de réglage (12), peut être amené en appui sur l'extrémité libre (14) de chacun de ces segments (9) de telle manière que les tétons de réglage (10) des segments (9) repoussent une partie de paroi du conduit souple (16) dans les rainures longitudinales (5) de l'embout de raccordement (2).

2. Dispositif selon la revendication 1, caractérisé en ce que le filetage de l'embout fileté (6), qui guide l'écrou de réglage (12), n'est disposé que sur la surface latérale extérieure de ceux des segments (9) qui ne sont pas pourvus d'un téton de réglage (10).

3. Dispositif selon la revendication 1 ou 2, caractérisé en ce que les rainures longitudinales (5) présentent une section transversale qui s'élargit en forme de V vers l'extérieur et en ce que les tétons de réglage (10) sont formés de manière à pouvoir être insérés dans leur rainure longitudinale (5) correspondante.

4. Dispositif selon la revendication 2 ou 3, caractérisé en ce que l'embout fileté (6) et/ou l'écrou de réglage (12) sont pourvus de moyens (15) qui, pour le moins, rendent plus difficile le dévissage complet de l'écrou de réglage (12) de l'embout fileté.
